# EUROPEAN PATENT SPECIFICATION

(11) **EP 2 150 816 B1**
(45) Date of publication and mention of the grant of the patent: **26.11.2014**
(21) Application number: 08705948.1
(22) Date of filing: 16.01.2008
(51) Int. Cl.: G01N 33/68

(54) **ASSESSING MAMMALS FOR VASCULAR DISEASES**
PRÜFUNG VON SÄUGETIEREN AUF GEFÄSSKRANKHEITEN
ÉVALUATION DE MAMMIFÈRES EN CAS DE MALADIES VASCULAIRES

(30) Priority: 16.01.2007 US 880551 P
(43) Date of publication of application: 10.02.2010
(73) Proprietor: MAYO FOUNDATION FOR MEDICAL EDUCATION AND RESEARCH, Rochester, MN 55905 (US)
(72) Inventor: KHOSLA, Sundeep, Rochester, Minnesota 55902 (US); LERMAN, Amir, Rochester, Minnesota 55902 (US); MOEDDER, Ulrike, Rochester, Minnesota 55902 (US); GOESSL, Mario, Rochester, Minnesota 55902 (US)
(74) Representative: Grund, Martin
(86) International application number: PCT/US2008/051151
(87) International publication number: WO 2008/089226

(56) References cited:
- GABBASOV Z A ET AL: "Detection of Circulating Stromal Stem Cells with Osteogenic Potential in the Blood of Coronary Patients by Laser Flow Cytometry", BULLETIN OF EXPERIMENTAL BIOLOGY AND MEDICINE, KLUWER ACADEMIC PUBLISHERS-PLENUM PUBLISHERS, NE, vol. 139, no. 2, 1 February 2005 (2005-02-01), pages 266-268, XP019217979, ISSN: 1573-8221, DOI: 10.1007/S10517-005-0266-6
- EGHBALI-FATOURECHI ET AL: "Characterization of circulating osteoblast lineage cells in humans", BONE, PERGAMON PRESS., OXFORD, GB, vol. 40, no. 5, 4 January 2007 (2007-01-04), pages 1370-1377, XP022025441, ISSN: 8756-3282, DOI: 10.1016/J.BONE.2006.12.064
- GOSSL MARIO ET AL: "Expression of osteocalcin by circulating endothelial progenitor cells predicts endothelial dysfunction or structural coronary artery disease", CIRCULATION, vol. 116, no. 16, Suppl. S, October 2007 (2007-10), pages 135-136, XP002666802, & 80TH ANNUAL SCIENTIFIC SESSION OF THE AMERICAN-HEART-ASSOCIATION; ORLANDO, FL, USA; NOVEMBER 04 -07, 2007 ISSN: 0009-7322
- MOEDDE U I ET AL: "Expression of osteocalcin by circulating endothelial progenitor cells predicts endothelial dysfunction or structural coronary artery disease", JOURNAL OF BONE AND MINERAL RESEARCH, vol. 22, no. Suppl. 1, September 2007 (2007-09), page S73, XP002666803, & 29TH ANNUAL MEETING OF THE AMERICAN-SOCIETY-FOR-BONE-AND-MINERAL-RESE ARCH; HONOLULU, HI, USA; SEPTEMBER 16 -19, 2007 ISSN: 0884-0431
- CAROLINE SCHMIDT-LUCKE ET AL: 'Reduced number of circulating endothelial progenitor cells predicts future cardiovascular events' CIRCULATION vol. 111, 2005, pages 2981 - 2987, XP002531336
- FREDERICK M. RAUSCHER ET AL: 'Aging, progenitor cell exhaustion, and atherosclerosis' CIRCULATION vol. 108, 2003, pages 457 - 463, XP002434091
- JONATHAN M. HILL ET AL: 'Circulating endothelial progenitor cells, vascular function, and cardiovascular risk' THE NEW ENGLAND JOURNAL OF MEDICINE vol. 348, 2003, pages 593 - 600, XP002980439
- MARIO GOSSL ET AL: 'Osteocalcin expression by circulating endothelial progenitor cells in patients with coronary atherosclerosis' JOURNAL OFTHE AMERICAN COLLEGE OF CARDIOLOGY vol. 52, no. 16, 2008, pages 1314 - 1325, XP025565416

## Description

### BACKGROUND

### 1. Technical Field

This document relates to in vitro methods involved in assessing mammals for vascular disease. For example, this document provides methods for using endothelial progenitor cells expressing a bone-related polypeptide (e.g., an osteocalcin polypeptide) as markers for vascular diseases (e.g., atherosclerosis).

### 2. Background Information

Vascular diseases such as coronary artery disease are common types of heart diseases and leading causes of morbidity and mortality. Coronary artery disease occurs when the arteries that supply blood to the heart muscle (coronary arteries) become hardened and narrowed due to a buildup of plaque on the inner walls or lining of the arteries (a process called atherosclerosis). Blood flow to the heart can be reduced as plaque narrows the coronary arteries. This can decrease oxygen supply to the heart muscle and can cause angina and heart attack. Over time, coronary artery disease can weaken heart muscle and contribute to heart failure and arrhythmias.

Analysis of blood by flow cytofluorometry has been described by Gabbasov et al. (Bulletin of Experimental Biology and Medicine 2005, 139(2):266-268). Eghbali-Fatourechi et al. (Bone 2007, 40(5):1370-1377) describe that there are circulating cells expressing CD34 and osteocalcin. However, none of the documents discloses diagnosis of vascular disease by determining CD34, KDR and a bone related peptide.

### SUMMARY

This document provides in vitro methods involved in assessing mammals for vascular disease (e.g., coronary artery disease). For example, this document provides methods for using circulating endothelial progenitor cells expressing a bone-related polypeptide (e.g., an osteocalcin polypeptide) as markers for vascular diseases (e.g., atherosclerosis).

In general, one aspect of this document features a in vitro method for assessing a mammal for vascular disease. The method comprises, or consists essentially of, determining whether or not the mammal contains an elevated level of circulating CD34+/KDR+ endothelial progenitor cells expressing a bone-related polypeptide, where the presence of the elevated level indicates that the mammal has vascular disease. The mammal can be a human. The vascular disease can be early coronary atherosclerosis. The vascular disease can be late coronary atherosclerosis. The circulating CD34+/KDR+ endothelial progenitor cells can be circulating CD133+/CD34+/KDR+ endothelial progenitor cells. The circulating CD34+/KDR+ endothelial progenitor cells can be circulating CD 133-/CD34+/KDR+ endothelial progenitor cells. The method can comprise classifying the mammal as having vascular disease when the mammal contains the elevated level. The method can comprise classifying the mammal as not having vascular disease when the mammal lacks the elevated level. The method can comprise recording that the mammal has vascular disease when the mammal contains the elevated level. The method can comprise recording that the mammal does not have vascular disease when the mammal lacks the elevated level. The bone-related polypeptide can be an osteocalcin polypeptide, an alkaline phosphatase polypeptide, a Stro-1 polypeptide, or an osteonectin polypeptide.

Unless otherwise defined, all technical and scientific terms used herein have the same meaning as commonly understood by one of ordinary skill in the art to which this invention pertains. In case of conflict, the present specification, including definitions, will control.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

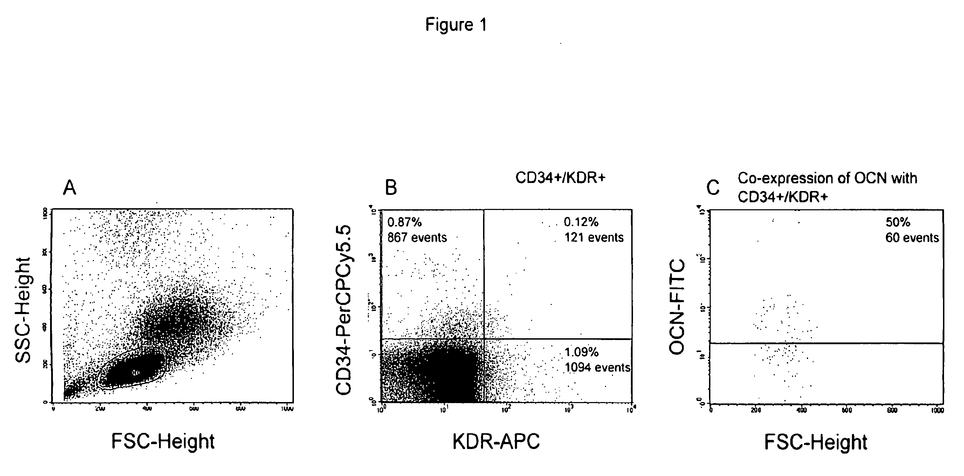

Figure 1 contains representative flow cytometry results. Panel A depicts the forward/side scatter and the gates used in the analysis. Panel B depicts the staining with the CD34- and KDR-specific antibodies, with the thresholds set based on the appropriate isotype controls. It was set such that < 0.5% of the cells were positive with the isotype controls, and the signal of the isotype was subtracted from the signal due to the specific antibodies. Panel C depicts further staining of the CD34+/KDR+ cells with the OCN antibody.

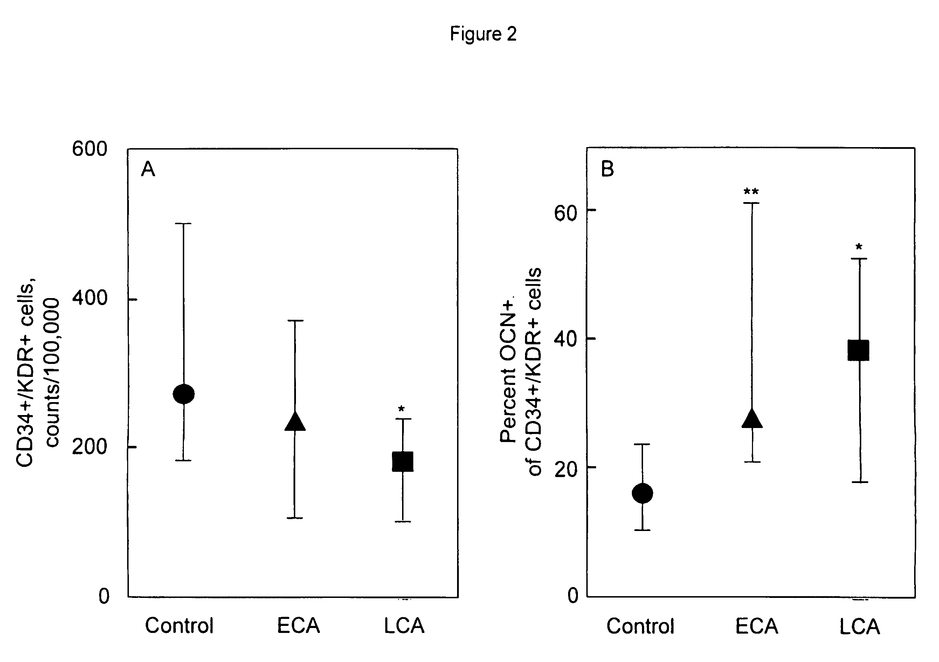

Figure 2 contains graphs plotting (A) CD34+/KDR+ cells, expressed as absolute counts per 100,000 counts, and (B) percent OCN+ of CD34+/KDR+ cells in control subjects (circles), for patients with early coronary atherosclerosis (ECA, triangles), and for patients with late coronary atherosclerosis (LCA, squares). *P < 0.05 and **P < 0.01 compared with control subjects. The median values and IQRs are shown.

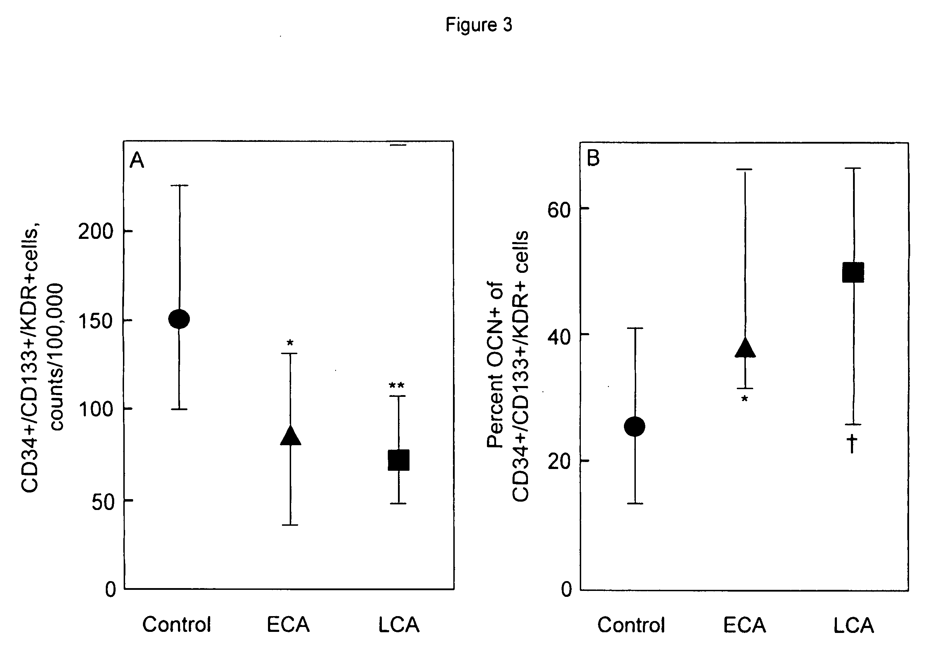

Figure 3 contains graphs plotting (A) CD34+/CD133+/KDR+ cells, expressed as absolute counts per 100,000 counts, and (B) percent OCN+ of CD34+/CD133+/KDR+ cells, in control subjects (circles), in patients with early coronary atherosclerosis (ECA, triangles), and in patients with late coronary atherosclerosis (LCA, squares). *P < 0.05, **P < 0.01, and ^{†}P = 0.069 compared with control subjects. The median values and IQRs are shown.

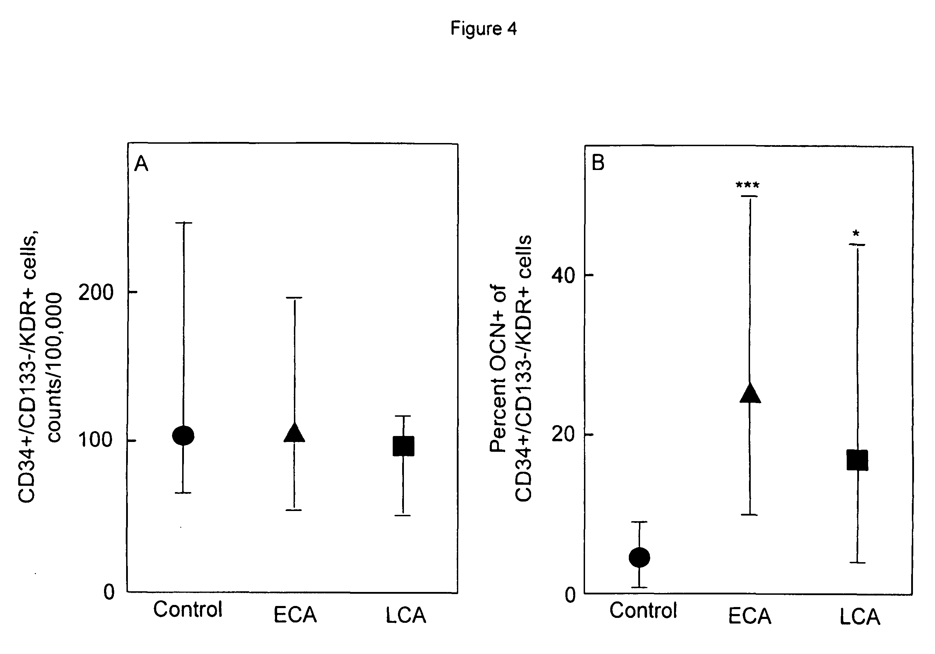

Figure 4 contains graphs plotting (A) CD34+/CD133-/KDR+ cells, expressed as absolute counts per 100,000 counts, and (B) percent OCN+ of CD34+/CD133-/KDR+ cells in control subjects (circles), in patients with early coronary atherosclerosis (ECA, triangles), and in patients late coronary atherosclerosis (LCA, squares). *P < 0.05 and ***P < 0.001 compared with control subjects. The median values and IQRs are shown.

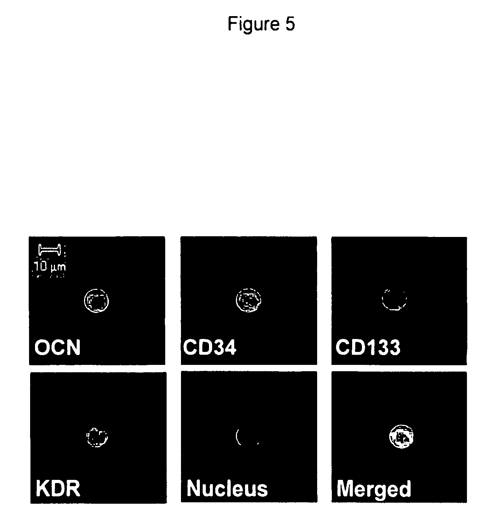

Figure 5 contains confocal microscopy photographs of a cell stained with antibodies to OCN (visible green stain), CD133 (visible red stain), CD34 (visible brown stain), KDR (visible blue-green stain) or stained with a stain for nuclei (DAPI; visible blue stain). Figure 3 also contains a merged image, which represents all the colors.

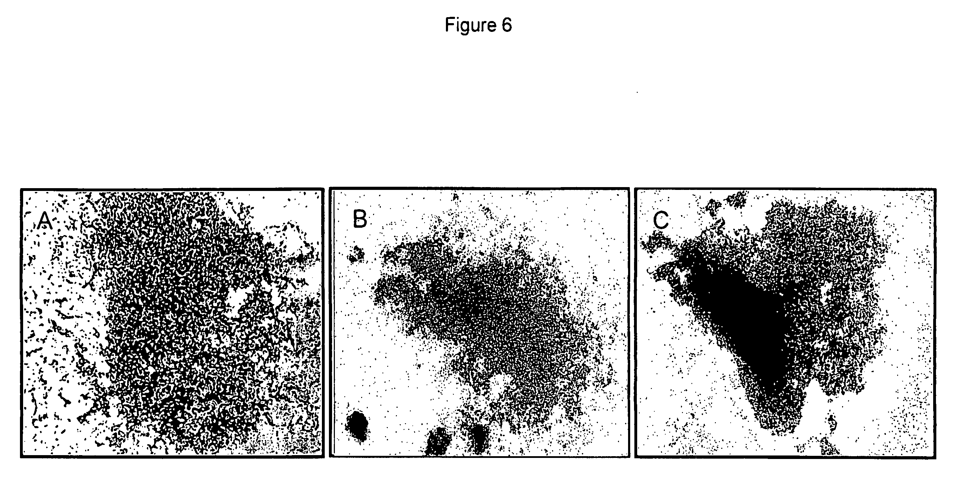

Figure 6 contains three photographs of in vitro mineralization assay results of CD34+ cells from an anonymous blood donor (A) using von Kossa staining and (B) using Alizarin Red staining, and (C) of PBMNCs from a patient with LCA (Alizarin Red stain). In these cultures, cells were exposed to Ca²⁺ for 5 days followed by 21 days of culture in osteoblast differentiation medium. All photographs are at x 4 magnification.

### DETAILED DESCRIPTION

This document provides in vitro methods related to assessing mammals for vascular disease (e.g., coronary artery disease). For example, the methods provided herein can be used to determine whether or not a mammal with or without a history of a coronary event (e.g., myocardial infarction, acute coronary syndrome, or a previous need for revascularization) has vascular disease or is likely to develop vascular disease. The mammal can be a human, non-human primate, goat, horse, cow, pig, dog, or cat. In some cases, the mammal can be a healthy human with no history of a heart problem. A mammal can be classified as having vascular disease or as being likely to develop vascular disease by assessing the level of circulating endothelial progenitor cells (EPCs) expressing a bone-related polypeptide in the mammal. For example, a mammal found to have an elevated level of circulating EPCs expressing a bone-related polypeptide (e.g., an osteocalcin polypeptide) can be classified as having vascular disease or as being likely to develop vascular disease. Examples of bone-related polypeptides include, without limitation, osteocalcin, alkaline phosphatase, Stro-1, and osteonectin polypeptides.

The term "elevated" as used herein with reference to circulating EPCs expressing a bone-related polypeptide refers to any level of such EPCs that is greater than either the level of such EPCs present in a control sample or the average level of such EPCs present in samples from a population of normal healthy mammals (e.g., a population of humans known to be free of vascular disease). For example, when assessing a particular mammal for vascular disease, the level of circulating EPCs expressing a bone-related polypeptide present in a blood sample from that mammal can be compared to the level of circulating EPCs expressing a bone-related polypeptide present in blood samples obtained from a population of mammals known to be free of vascular disease. Any population size can be used to determine the average level of circulating EPCs expressing a bone-related polypeptide present in samples from a population of normal healthy mammals. For example, a population of 2, 3, 4, 5, 10, 15, 20, 25, 30, 40, 50, or more humans free of vascular disease can be used to determine the average level of circulating EPCs expressing a bone-related polypeptide present in samples from a population of normal healthy mammals. An elevated level of circulating EPCs expressing a bone-related polypeptide can be 5, 10, 20, 30, 50, or more percent higher than that level found in a control sample or the average level of circulating EPCs expressing a bone-related polypeptide present in samples from a population of normal healthy mammals. In some cases, an elevated level of circulating EPCs expressing a bone-related polypeptide can be 1.1, 1.2, 1.5, 2, 3, 4, 5, 10, 50, 100, or more fold higher than that level present in a control sample or the average level of circulating EPCs expressing a bone-related polypeptide present in samples from a population of normal healthy mammals.

In some cases, an elevated level of circulating EPCs expressing a bone-related polypeptide (e.g., an osteocalcin polypeptide) in a human can be any level greater than about 35 percent (e.g., greater than about 40, 45, 50, 55, or 60 percent), where the percentage is the percent of CD133+/CD34+/KDR+ EPCs that are osteocalcin positive. In such cases, the range for an elevated can be between 35 and 60 percent (e.g., between 40 and 60 percent, between 45 and 60 percent, between 40 and 55 percent, or between 40 and 50 percent). In some cases, an elevated level of circulating EPCs expressing a bone-related polypeptide (e.g., an osteocalcin polypeptide) in a human can be any level greater than about 15 percent (e.g., greater than about 20, 25, 30, 35, 40, 45, 50, 55, or 60 percent), where the percentage is the percent of CD133-/CD34+/KDR+ EPCs that are osteocalcin positive. In such cases, the range for an elevated can be between 15 and 60 percent (e.g., between 20 and 60 percent, between 20 and 50 percent, between 20 and 40 percent, or between 15 and 40 percent).

In some cases, a reference chart can be used to determine whether or not a particular level of circulating EPCs expressing a bone-related polypeptide in a sample is elevated, normal, or reduced. For example, a reference chart can contain the normal range of circulating EPCs expressing a bone-related polypeptide found in samples from mammals with or without vascular disease. Using this reference chart, any level of circulating EPCs expressing a bone-related polypeptide measured in a sample can be classified as being an elevated level, a normal level, or a reduced level.

As described herein, the presence of an elevated level of circulating EPCs expressing a bone-related polypeptide (e.g., an osteocalcin polypeptide) in a mammal's sample can indicate that the mammal has vascular disease or is likely to develop a vascular disease. In the case of humans, an osteocalcin polypeptide can be a human osteocalcin polypeptide such as a polypeptide having the amino acid sequence set forth in Genbank GenInfo Identifier (GI) No. 62865480; an alkaline phosphatase polypeptide can be a human alkaline phosphatase polypeptide such as a polypeptide having the amino acid sequence set forth in Genbank GI No. 178427; and an osteonectin polypeptide can be a human osteonectin polypeptide such as a polypeptide having the amino acid sequence set forth in Genbank GI No. 338324. A Stro-1 polypeptide can be a human Stro-1 polypeptide such as a human Stro-1 polypeptide having the ability to bind to an anti-STRO-1 antibody. An anti-STRO-1 antibody can be obtained from the Developmental Studies Hybridoma Bank (The University of lowa, Department of Biological Studies, lowa City, IA 52242; catalog number: STRO-1). In the case of non-human mammals, an osteocalcin polypeptide can be a corresponding osteocalcin polypeptide; an alkaline phosphatase polypeptide can be a corresponding alkaline phosphatase polypeptide; a Stro-1 polypeptide can be a corresponding Stro-1 polypeptide; and an osteonectin polypeptide can be a corresponding osteonectin polypeptide. For example, in the case of *Mus musculus,* an osteocalcin polypeptide can be a mouse osteocalcin polypeptide such as a polypeptide having the amino acid sequence set forth in Genbank GI No. 88156, 88157, or 338324.

The level of circulating EPCs expressing a bone-related polypeptide can be determined using, for example, a blood sample obtained from a mammal. Any appropriate method can be used to obtain a sample from a mammal. For example, blood samples can be obtained via venous puncture techniques using a needle and syringe.

Any appropriate method can be used to determine the level of EPCs expressing a bone-related polypeptide in a sample. For example, the level of EPCs expressing an osteocalcin polypeptide can be determined by determining the percent of CD34+/KDR+ EPCs (e.g., CD133+/CD34+/KDR+ EPCs or CD133-/CD34+/KDR+ EPCs) that are osteocalcin positive. Immunological-based techniques such as flow cytometry can be used to determine the number of cells within a sample that express a particular polypeptide (e.g., CD133, CD34, KDR, osteocalcin, alkaline phosphatase, Stro-1, and osteonectin).

Once identified as having vascular disease or as being likely to develop a vascular disease, the mammal can begin treatment or can undergo additional testing to confirm or evaluate vascular disease. For example, a mammal classified as having vascular disease as described herein can undergo diagnostic tests such as coronary angiography, coronary blood flow measurements, or intravascular ultrasound to confirm the existence or severity of vascular disease. In some cases, a mammal classified as having vascular disease as described herein can initiate one or more treatments designed to reduce the likelihood of experiencing a coronary condition. Such treatments can include, without limitation, administering agents such as statins (e.g., simvastatin or pravastatin), blood-thinning agents (e.g., warfarin, enoxaparin sodium injection, or aspirin), and cholesterol reuptake or absorption inhibitors (e.g., ezetimibe).

Disclosed are kits that can be used to assess vascular disease. For example, reagents used to determine the level of circulating EPCs expressing a bone-related polypeptide in a sample can be combined as an article of manufacture such as a kit. In one embodiment, a kit can contain reagents for the immunodetection of a bone-related polypeptide (e.g., an osteocalcin polypeptide) as well as reagents for the immunodetection of CD133, CD34, and/or KDR. For example, a kit can contain an anti-osteocalcin polypeptide antibody. In some cases, a kit can contain buffers, positive control samples (e.g., a solution containing a known amount of osteocalcin positive EPCs), or combinations thereof. The reagents within a kit can be housed together in various combinations or can be packaged in separate vials or containers. The kits also can include labels or packaging inserts setting out instructions for preparation and use. For example, a kit can contain a packaging insert describing that the level of circulating EPCs expressing a bone-related polypeptide (e.g., an osteocalcin polypeptide) can be used to assess the presence or absence of vascular disease.

The term "antibody" as used herein includes, without limitation, polyclonal, monoclonal, multi-specific, human, humanized, chimeric, and single-chain antibodies as well as antibody fragments having binding activity such as Fab fragments, F(ab') fragments, F(ab')2 fragments, Fd fragments, fragments produced by a Fab expression library, fragments comprising a VL or VH domain, and epitope binding fragments of any of the above. An antibody can be of any type (e.g., IgG, IgE, IgM, IgD, IgA, and IgY), class (e.g., IgG1, IgG2, IgG3, IgG4, IgA1, and IgA2), or subclass. In addition, antibodies can be from any animal including birds and mammals. For example, antibodies can be human, murine, rabbit, goat, guinea pig, camel, horse, or chicken.

An antibody can be naturally occurring, recombinant, or synthetic. Antibodies can be generated by any suitable method known in the art. For example, monoclonal antibodies can be prepared using a wide variety of techniques known in the art, including the use of hybridoma, recombinant, and phage display technologies, or a combination thereof. An antibody such as an antibody fragment can be produced by any means. For example, an antibody fragment can be enzymatically or chemically produced by fragmentation of an intact antibody. An antibody fragment can also be produced synthetically or recombinantly from a gene encoding the partial antibody sequence.

Once an antibody has been produced by an animal, chemically synthesized, or recombinantly expressed, it can be purified by any method known in the art. For example, an antibody can be purified by chromatography, centrifugation, or differential solubility. In some cases, an antibody can be fused to a sequence, such as a hexa-histidine peptide, for convenient purification of the fusion protein.

As described herein, methods for assessing a mammal for vascular disease based on the level of circulating EPCs expressing a bone-related polypeptide (e.g., an osteocalcin polypeptide) in the mammal are provided. Mammals that can be assessed for vascular disease include mammals that are adults. For example, a human between the ages of about 18 and 85 (e.g., 25, 30, 35, 40, 45, 50, 55, 60, 65, 70, 75, 80, 85, or 90 years of age) can be assessed for vascular disease. In addition, mammals that can be assessed for vascular disease include mammals that were or were not previously diagnosed with vascular disease, as well as mammals that have vascular disease, are suspected of having vascular disease, or are susceptible to developing vascular disease, such as mammals having a family history of a coronary condition.

The invention will be further described in the following examples, which do not limit the scope of the invention described in the claims.

### EXAMPLES

### Example 1 - Osteocalcin Expression by Circulating Endothelial Progenitor Cells in Patients with Coronary Atherosclerosis

### Study subjects

An institutional approved study was performed. All study subjects provided written, informed consent. A total of 59 consecutive patients who were undergoing coronary angiography and coronary endothelial function testing, and met the criteria outlined below for inclusion into the three study groups, were recruited. Patients with acute coronary syndromes (unstable angina or acute myocardial infarction), heart failure (ejection fraction < 50%), or severe renal or liver disease were excluded. The three groups were defined as follows: (1) control, patients without significant structural coronary lesions on angiography (< 30% stenosis) and normal endothelial function, as assessed by intra-coronary acetylcholine challenge (see below), n = 11; (2) early coronary atherosclerosis (ECA), defined by the absence of significant structural coronary lesions on angiography but with abnormal endothelial function, n = 19; and (3) late coronary atherosclerosis (LCA), defined as patients with severe, 3-vessel CAD (> 50% stenosis in three or more major epicardial arteries), n = 29.

### Coronary angiography and invasive endothelial function testing

Patients underwent coronary angiography using standard clinical protocols. Arterial blood (20 mL) was drawn from the aortic catheter before cardiac catheterization for flow cytometry and biochemical analyses (see below). After diagnostic angiography, subjects without significant structural coronary lesions underwent assessment of endothelium-dependent coronary vasoreactivity, as described elsewhere (Suwaidi et al., Am. J. Cardiol., 88:1300-3 (2001); Hasdai et al., Circulation, 96:3390-5 (1997); Lerman and Zeiher, Circulation, 111:363-8 (2005); and Suwaidi et al., Circulation, 101:948-54 (2000)). In brief, a Doppler guidewire (Flowire, Volcano Inc) within a coronary-infusion catheter (Ultrafuse, SciMed Life System) was positioned into the mid-portion of the left anterior descending coronary artery. Acetylcholine at increasing concentrations of 10⁻⁶, 10⁻⁵, and 10⁻⁴ M (three minutes per dose) was infused into the left anterior descending coronary artery to assess endothelium-dependent vasoreactivity. Hemodynamic data, Doppler measurements, and a coronary angiogram were obtained after each infusion. Coronary artery diameter was measured by an independent investigator in the segment 5 mm distal to the tip of the Doppler wire using a computer-based image analysis system. Average peak velocity (APV) was derived from the Doppler flow velocity spectra and coronary blood flow (CBF) was determined as π(coronary artery diameter/2)² X (APV/2). According to previous studies, microvascular endothelial dysfunction was defined as an increase in CBF of < 50%, and epicardial endothelial dysfunction was defined as a decrease in epicardial coronary artery diameter of more than 20% in response to the maximal dose of acethylcholine (10⁻⁴ M) *(*Suwaidi et al., Am. J. Cardiol., 88:1300-3 (2001); Hasdai et al., Circulation, 96:3390-5 (1997); Lerman and Zeiher, Circulation, 111:363-8 (2005); and Suwaidi et al., Circulation, 101:948-54 (2000)). Endothelium-independent microvascular function was determined by the coronary flow reserve (CFR), which is the ratio of the APV at maximal hyperemia (induced by intracoronary adenosine (18-60 µg)) to the APV at baseline.

### Flow cytometry

Mononuclear cells were isolated using a Ficoll density gradient, and immunofluorescent cell staining was performed using the following fluorescent conjugated antibodies: CD34-PerCP Cy 5.5 (Beckton-Dickinson), CD133-phycoerythrin (PE) (Miltenyi Biotec GmbH), and kinase insert domain receptor KDR-APT (R & D Systems) and the appropriate isotype controls. In addition, OCN+ cells were identified using an anti-human OCN antibody (Santa Cruz Biotechnology) and a fluorescein isothiocyanate (FITC) secondary antibody (Jackson ImmunoResearch), as described elsewhere (Eghbali-Fatourechi et al., N. Engl. J. Med., 352:1959-66 (2005)). Cell fluorescence was measured immediately after staining (Becton Dickinson, FACS Calibur), and data were analyzed using CellQuest software (Becton Dickinson). A total of 150,000 events were counted, and final data were obtained within the lymphocyte gate. In all subjects, two complete sets of immunofluorescent cell staining were obtained, and the mean of the two data sets for each subject was used in the analysis. The person doing the cell analysis was not aware of the results of the patient classification.

### Confocal microscopy

For confocal microscopy, cells were stained for OCN-FITC and CD34-PerCP Cy 5.5 and isolated by an autoMACSTM cell sorter (Miltenyi Biotec GmbH) with the appropriate micro-beads. The cells were then co-stained with the CD133-PE and KDR-APC antibodies. Following fixation with 4% paraformaldehyde, the cells were mounted in ProLong® Gold antifade reagent with DAPI (Invitrogen) to stain the nucleus. Samples were then examined on an LSM510 confocal laser scanning microscope (Carl Zeiss, Inc) equipped with an Axiovert 100 M microscope stand and a C-Apochromat 63x/1.2 n.a. water immersion objective lens.

### Cell Culture

CD34+ cells isolated by MACS, as described above, or unsorted post-Ficoll PBMNCs were cultured in uncoated plastic culture plates in regular culture media (αMEM, 10% heat-inactivated FBS, 1% penicillin/streptomycin/amphotericin B (all from GIBCO/Invitrogen Corporation)) for 2 days and then were either maintained in regular culture medium or were changed to regular culture medium containing an additional 2.1 mM Ca2+ for 5 days (days 3-7) of culture. Subsequently, the Ca2+ was removed, and the cells were cultured in osteoblast differentiation medium (αMEM, 10% heat-inactivated FBS, 1% penicillin/streptomycin/amphotericin B plus 50 µg/mL ascorbic acid phosphate magnesium salt n-Hydrate (Wako), 10 mM β-glycerophosphate, 10⁻⁸ M 1,25-dihydroxyvitamin D3, 10⁻⁸ M dexamethasone (Sigma)) for an additional 21 days (days 8-28), and the presence of mineralization determined by Alizarin Red and Von Kossa staining.

### Biochemical assays

Serum lipids (cholesterol, HDL cholesterol, triglycerides) were measured using enzymatic colorimetry, and LDL cholesterol calculated from these parameters. High sensitivity C-reactive protein (hs-CRP) levels were measured using a latex particle-enhanced immunoturbidemetric assay on a Hitachi 912 automated analyzer.

### Statistical analyses

Pre-specified comparisons between the ECA or LCA patients and control subjects were made using the Wilcoxon rank-sum test for continuous variables and the Fisher's Exact test for categorical variables. All data were presented as median (inter-quartile (25th-75th percentile) range, IQR). Logistic regression models following log (base 2) transformation, where appropriate, of the percentage of CD34+/KDR+ cells, of CD34+/CD133+/KDR+ cells, and of CD34+/CD133-/KDR+ cells co-staining for OCN or of hs-CRP were used to assess the ability of these variables to predict coronary atherosclerosis (ECA or LCA) following adjustment for age, sex, statin use, diabetes, hypertension, and smoking. Model assumptions and conclusions were checked.

Receiver-operator characteristic (ROC) curve analysis was used to test the ability of the percentage of CD133-/CD34+/KDR+ cells co-staining for OCN, hs-CRP, or the Framingham risk score *(*Wilson et al., Circulation, 97:1837-47 (1998)) to predict coronary atherosclerosis (ECA or LCA), and a non-parametric test of correlated ROC curve areas (Hanley and McNeil, Radiology, 148:839-43 (1983)) was used to compare ROC curve areas. All data were presented as mean ± SEM.

### Resulis

The relevant clinical and biochemical data as well as the detailed cardiac catherterization data in the study subjects are shown in Table 1. Subjects with LCA were significantly older than the control subjects, and were predominantly male. However, subjects with ECA had an age and gender mix not significantly different from the control subjects. BMI was similar across groups, but a higher percentage of the LCA subjects were on statins as compared to control subjects. The prevalence of diabetes, hypertension, and smoking was virtually identical in the control and ECA subjects, but higher for all three parameters in the LCA compared with the control subjects. The Framingham score was non-significantly higher in the subjects with ECA and significantly higher in the subjects with LCA as compared to the control subjects. Blood lipids were similar in the ECA subjects compared to the control subjects, with the LCA subjects having lower total and HDL cholesterol levels as compared to the control subjects. While hs-CRP levels were significantly higher in the ECA as compared to control subjects, patients with LCA had hs-CRP levels that were similar to control subjects. Overall, therefore, while there were significant differences in the clinical characteristics of the LCA patients compared to control subjects, the ECA patients had clinical and biochemical features that were very similar to the control subjects, with the exception of higher hs-CRP levels in the ECA patients.

**Table 1. Clinical, biochemical, and cardiac catheterization data of the studv subjects.**

| | Control | ECA | LCA |
|---|---|---|---|
| N | 11 | 19 | 29 |
| Clinical data | | | |
| Age, years | 44 (42, 51) | 52 (43, 60) | 72 (63, 80)*** |
| Male, n (%) | 2 (18) | 8 (42) | 26 (90)*** |
| BMI, kg/m² | 27 (26, 32) | 30 (25, 35) | 29 (27, 33) |
| Statin use, n (%) | 4 (36) | 10 (53) | 24 (83)** |
| Diabetes, n (%) | 0 (0) | 0 (0) | 7 (24) |
| Hypertension, n (%) | 4 (36) | 6 (32) | 23 (79)* |
| Smoking, n (%) | 2 (18) | 4 (21) | 18 (62)* |
| Framingham score | 0 (-2, 4) | 4 (1, 6) | 7 (5, 9)*** |
| Biochemical data | | | |
| Total cholesterol, mg/dL | 187 (160, 240) | 194 (169, 241) | 154 (129, 184)* |
| HDL cholesterol, mg/dL | 57 (53, 82) | 61 (44, 75) | 45 (40, 53)** |
| Triglycerides, mg/dL | 108 (53, 125) | 97 (69, 143) | 105 (88, 169) |
| LDL cholesterol, mg/dL | 105 (92, 123) | 113 (96, 136) | 78 (58, 103) |
| hs-CRP, mg/L | 1.0 (0.3, 2.6) | 2.0 (1.3, 4.0)* | 0.6 (0.1, 1.4) |
| Cardiac catheterization data | | | |
| Mean coronary artery stenosis based on all angiographically identified stenoses, % | 20 (5, 25) | 20 (10, 22) | 70 (60, 78)*** |
| Mean coronary artery stenosis based on coronary arteries with >50% stenosis, % | ND | ND | 90 (86, 94) |
| Microvascular endothelial function (Δ coronary blood flow to 10⁻⁴ M acetylcholine, %) | 105.8 (64.8, 187.0) | -1.6 (-47.2, 17.0)*** | ND |
| Epicardial endothelial function (Δ coronary diameter to 10⁻⁴ M acetylcholine, %) | 0.0 (-11.4, 6.8) | -25.0 (-48.1, -15.3)*** | ND |
| Endothelium-independent microvascular function | 2.8 (2.6, 3.7) | 2.8 (2.1, 3.4) | ND |
| (coronary flow reserve) | | | |

| | | | |
|---|---|---|---|
| ECA, early coronary atherosclerosis LCA, late coronary atherosclerosis Data are median (IQR) ND, not determined *P < 0.05, **P < 0.01, ***P < 0.001 vs. Control | | | |

Table 1 also provides the detailed cardiac catheterization data in the study subjects. Based on the definition of the groups, the ECA subjects did not have significant structural coronary artery disease, whereas the LCA patients had a mean coronary artery stenosis of 90%, when calculated based on coronary arteries with at least a 50% stenosis (Werner et al., N. Engl. J. Med., 353:999-1007 (2005) and Holmes et al., JAMA, 295:1264-1273 (2006)). Sixty-six percent of the patients in the LCA group had 3-vessel disease, and 34 % had 2-vessel disease. Parameters of epicardial and microvascular endothelial function were significantly different in the ECA as compared to the control subjects.

Flow cytometry was performed using antibodies to the EPC markers and OCN, and Figure 1 provides a representative example of the flow analysis for CD34, KDR, and OCN. In preliminary experiments, it was determined that the vast majority of the CD34+/KDR+ cells were in the small lymphocyte gate (Figure 1A), as has also been noted by other investigators (Werner et al., N. Engl. J. Med., 353:999-1007 (2005) and Vasa et al., Circ. Res., 89:e1-e7 (2001)). Figure 1B depicts the flow cytometry analysis for CD34+/KDR+ cells, and Figure 1C depicts the further analysis of these cells with the OCN antibody.

Figure 2 depicts the analysis of peripheral blood mononuclear cells for these markers in the 3 groups of subjects. The number of CD34+/KDR+ cells were reduced (by about 30%) in the LCA, but not the ECA, subjects as compared to the control subjects (Figure 2A). By contrast, the percentage of the CD34+/KDR+ cells co-staining for OCN was significantly increased in both the ECA and LCA patients (by 1.7- and 2.5-fold, respectively, Figure 2B) compared with the control subjects.

To gain further insight into these changes in CD34+/KDR+ cells in the vascular disease groups, sub-populations of the CD34+/KDR+ cells were examined based on CD133 expression. The number of CD34+/CD133+/KDR+ cells were significantly reduced (by ~50%) in both the ECA and LCA patients as compared to the control subjects (Figure 3A). As for the total CD34+/KDR+ cells, the percent OCN+ of CD34+/CD133+/KDR+ cells were also increased in the ECA and LCA patients (by 1.5-and 2-fold, respectively, Figure 3B) compared with the control subjects.

In contrast to the reduction in the number of CD34+/CD133+/KDR+ cells in the vascular disease groups, the number of CD34+/CD133-/KDR+ cells did not differ between groups (Figure 4A). However, the percent OCN+ of CD34+/CD133-/KDR+ cells was markedly higher in the ECA and LCA patients (by 8.3- and 5.3-fold, respectively, Figure 4B) compared with the control subjects.

Figure 5 provides evidence, using confocal microscopy, that each of the antibodies used herein was binding to the surface of the cell. The relatively small size (about 10 µm) of these cells, as represented by a cell positive with all four antibodies (CD34, CD133, KDR, and OCN), was noted.

The following was assessed to determine whether peripheral blood CD34+ cells were capable of mineralization *in vitro.* For these experiments, CD34+ cells isolated from anonymous blood donors were used. When cultured on uncoated plastic plates, these cells failed to adhere. By 21 days in osteoblast differentiation medium, the culture plates contained no visible cells. Reasoning that local calcium deposition in the vasculature due either to cell death or vascular micocalcifications might induce these cells to adhere, the CD34+ cells were exposed to an additional 2.1 mM of extracellular Ca²⁺ only for 5 days at the beginning of culture (days 3-7). Under these conditions, peripheral blood CD34+ cells were found to adhered routinely to the plastic dishes and, when placed in osteogenic differentiation medium for an additional 21 days, to form mineralized nodules, as assessed either by Von Kossa (Figure 6A) or Alizarin Red (Figure 6B) staining. Note that in these assays, from days 8 to 28 (21 days of differentiation media) the cells were in osteoblast differentiation medium without additional calcium. Since sufficient numbers of cells from the patients undergoing cardiac catheterization were not obtained in order to specifically isolate CD34+ cells for culture, PBMNCs either from normal subjects (n = 3) or patients with LCA (n = 3) were tested to determine whether they could be induced to mineralize under similar culture conditions. PBMNCs from normal subjects, who had percent OCN+ of CD34+ cells of 4.2%, 9.9%, and 12.7%, respectively, did not form mineralized colonies, without or with calcium exposure. Of the LCA patients, PBMNCs from the subject with the highest percent OCN+ of CD34+ cells (31.2%) were able to form mineralized deposits in the presence, but not in the absence of the brief 5 day exposure to calcium (Figure 6C), whereas PBMNCs from the other 2 LCA patients who had lower percent OCN+ of CD34+ cells (18.3% and 24.9%) failed to mineralize under these conditions.

The following was performed to determine the ability of OCN+ cells to predict coronary atherosclerosis. Since by the design of the study (consecutive recruitment of subjects referred for cardiac catheterization), one could not control the age, gender, statin use, or other risk factors (diabetes, hypertension, smoking) in the study subjects, logistic regression models were performed using all subjects (normal and coronary atherosclerosis). The ability of the percent OCN+ of CD34+/KDR+, the percent OCN+ of CD34+/CD133+/KDR+, and the percent OCN+ of CD34+/CD133-/KDR+ cells to predict coronary atherosclerosis (ECA or LCA) was tested in multiple logistic regression models that included age, gender, statin use, diabetes, hypertension, and smoking. Following adjustment for these other factors, the percent OCN+ of CD34+/KDR+ or the percent OCN+ of CD34+/CD133+/KDR+ cells were only of borderline significance (P = 0.07 and 0.120, respectively) as predictors of coronary atherosclerosis. However, the percent OCN+ of CD34+/CD133-/KDR+ cells remained significant (P = 0.022) as predictors of coronary atherosclerosis in these models, with an odds ratio (95% confidence interval) following adjustment for all of these factors of 7.3 (1.3-39.8). Since in these models a log₂ transformation of the percent OCN+ of CD34+/CD133-/KDR+ cells was used, the odds ratio represents the increase in risk associated with a doubling in the percentage of these cells. Note, however, that due to the relatively small number of subjects, the 95% confidence interval for this estimate was fairly wide, although clearly did not include one.

Finally, ROC analyses were performed to further assess the ability of the robust markers, the percent OCN+ of CD34+/CD133-/KDR+ cells to predict coronary atherosclerosis (ECA or LCA). This was placed in the context of a similar analysis using the currently available marker, hs-CRP (Table 2). As is evident, both for unadjusted and for age-adjusted models, the percent OCN+ of CD34+/CD133-/KDR+ cells performed better than hs-CRP as a predictor of coronary atherosclerosis.

**Table 2. Unadjusted and age-adjusted areas under the curve (AUC) for receiver operator characteristic (ROC) curves for prediction of coronary atherosclerosis (early coronary atherosclerosis (ECA) or late coronary atherosclerosis (LCA) combined), using the percent OCN+ of CD34+/CD133-/KDR+ cells or hs-CRP levels as predictors.**

| Predictor | Unadjusted AUC (95% confidence interval) | Age-adjusted AUC (95% confidence interval) |
|---|---|---|
| hs-CRP^{a} | 0.51 (0.32-0.69) | 0.85 (0.75-0.95) |
| % OCN+ of CD34+/CD133-/KDR+ cells^{a} | 0.80 (0.68-0.93)* | 0.95 (0.89-1.00)^{†} |

| | | |
|---|---|---|
| ^{a}log₂ used *P < 0.05 and ^{†}P = 0.054 vs. corresponding AUC for hs-CRP | | |

The results provided herein demonstrate that patients with ECA, characterized by coronary endothelial dysfunction, or those with established LCA, characterized by severe, 3-vessel CAD, have a significantly greater percentage of cells co-staining for OCN both in the population containing intermediate (CD133+/CD34+/KDR+) and more mature (CD133-/CD34+/KDR+) EPCs as compared to control subjects with normal endothelial function and no structural CAD. The results provided herein also demonstrate that cells co-staining for OCN both in the population containing intermediate (CD133+/CD34+/KDR+) and more mature (CD133-/CD34+/KDR+) EPCs can be used as markers for vascular disease. For example, the results provided herein demonstrate that the percent OCN+ of CD133-/CD34+/KDR+ cells can serve as a peripheral marker of coronary atherosclerosis. From a diagnostic standpoint, these cells appeared to be more consistent predictors of ECA or LCA than either hs-CRP or the Framingham score. The relatively poor predictive ability of hs-CRP for LCA may be due to the fact that most of these patients were on statins, which can lower CRP levels (Lloyd-Jones et al., Ann. Intern. Med., 145:35-42 (2006)), but statin use did not seem to prevent the increase in the percent OCN+ of CD133-/CD34+/KDR+ cells observed in the LCA patients.

A strength of the results provided herein is that the subjects defined as controls, having ECA with endothelial dysfunction, or LCA with 3-vessel CAD, were classified using coronary angiography and the gold standard for assessment of endothelial function, intra-coronary infusion of acetylcholine. Thus, misclassification of patients among these categories is extremely unlikely.

In summary, the results provided here demonstrate that a higher percentage of circulating cell populations containing EPCs co-stain for OCN in patients with early or late coronary atherosclerosis as compared to control subjects with normal endothelial function and no structural CAD. These results suggest that the activation of an osteogenic program by EPCs may induce vascular calcification, as opposed to non-calcifying vascular repair. Additionally, the same pro-inflammatory factors involved in the pathogenesis of osteoporosis may lead to the expression of an osteogenic phenotype by endothelial lineage cells, providing a potential mechanism for the link between osteoporosis and vascular calcifications.

### Example 2 - Gradient of EPCs

In 12 patients with coronary endothelial dysfunction, blood samples were obtained simultaneously from the ostium of the left main coronary artery and the coronary sinus. There were significantly lower percentages of osteogenic EPCs (by (mean ± SEM]), 21 ± 7 %), particularly in the population containing relatively late EPCs, in the samples obtained from the coronary sinus as compared to the samples that were obtained from the left main coronary artery. These results suggest that there is a consumption of EPCs which are OCN+ across the coronary circulation in patients with early coronary disease.

### Example 3 - Bone-related markers

The following was performed to demonstrate that bone-related polypeptides other than OCN can be used to co-stain EPCs and thus identify patients with vascular disease. The mRNA for alkaline phosphatase (AP) was examined in CD34+/OCN+ cells versus CD34+/OCN- cells. The mRNA for AP was below the limit of detection for the assay in the CD34+/OCN- cells, but easily measurable in the CD34+/OCN+ cells. Based on the sensitivity of the assay, it was estimated that an approximately 1000-fold enrichment for the AP mRNA in the CD34+/OCN+ as compared to the CD34+/OCN- cells. Qualitatively similar results were obtained in two additional samples. These results demonstrate that not just OCN, but other bone related polypeptides (e.g., AP) can be used to co-stain EPCs and thus identify patients with vascular disease.

## Claims

1. An in vitro method for assessing a mammal for vascular disease, wherein said method comprises determining whether or not a sample from said mammal contains an elevated level of circulating CD34+/KDR+ endothelial progenitor cells expressing a bone-related polypeptide, wherein the presence of said elevated level indicates that said mammal has vascular disease.

2. The method of claim 1, wherein said mammal is a human.

3. The method of claim 1, wherein said vascular disease is early coronary atherosclerosis.

4. The method of claim 1, wherein said vascular disease is late coronary atherosclerosis.

5. The method of claim 1, wherein said circulating CD34+/KDR+ endothelial progenitor cells are circulating CD133+/CD34+/KDR+ endothelial progenitor cells.

6. The method of claim 1, wherein said circulating CD34+/KDR+ endothelial progenitor cells are circulating CD133-/CD34+/KDR+ endothelial progenitor cells.

7. The method of claim 1, wherein said method comprises classifying said mammal as having vascular disease when said mammal contains said elevated level.

8. The method of claim 1, wherein said method comprises classifying said mammal as not having vascular disease when said mammal lacks said elevated level.

9. The method of claim 1, wherein said method comprises recording that said mammal has vascular disease when said mammal contains said elevated level.

10. The method of claim 1, wherein said method comprises recording that said mammal does not have vascular disease when said mammal lacks said elevated level.

## Patentansprüche

1. Ein *in vitro*-Verfahren zur Prüfung auf eine Gefäßerkrankung in einem Säugetier, wobei jenes Verfahren das Bestimmen umfasst, ob eine Probe aus dem Säugetier einen erhöhten Spiegel zirkulierender CD34+/ KDR+ endothelialer Vorläuferzellen, die ein Knochen-ähnliches Polypeptid exprimieren, enthält oder nicht, wobei die Anwesenheit jener erhöhten Spiegel anzeigt, dass jenes Säugetier eine Gefäßerkrankungen hat.

2. Das Verfahren nach Anspruch 1, wobei jenes Säugetier ein Mensch ist.

3. Das Verfahren nach Anspruch 1, wobei jene Gefäßerkrankung frühe koronare Atherosklerose ist.

4. Das Verfahren nach Anspruch 1, wobei jene Gefäßerkrankung späte koronare Atherosklerose ist.

5. Das Verfahren nach Anspruch 1, wobei jene zirkulierenden CD34+/KDR+ endothelialen Vorläuferzellen zirkulierende CD133+/CD34+/KDR+ endotheliale Vorläuferzellen sind.

6. Das Verfahren nach Anspruch 1, wobei jene zirkulierenden CD34+/KDR+ endothelialen Vorläuferzellen zirkulierende CD133-/CD34+/KDR+ endotheliale Vorläuferzellen sind.

7. Das Verfahren nach Anspruch 1, wobei jenes Verfahren das Klassifizieren jenes Säugetiers umfasst, dass es eine Gefäßerkrankung hat, wenn jenes Säugetier jene erhöhten Spiegel aufweist.

8. Das Verfahren nach Anspruch 1, wobei jenes Verfahren das Klassifizieren jenes Säugetiers umfasst, dass es keine Gefäßerkrankung hat, wenn jenem Säugetier jene erhöhten Spiegel fehlen.

9. Das Verfahren nach Anspruch 1, wobei jenes Verfahren das Aufzeichnen umfasst, dass jenes Säugetier eine Gefäßerkrankung hat, wenn jenes Säugetier jene erhöhten Spiegel aufweist.

10. Das Verfahren nach Anspruch 1, wobei jenes Verfahren das Aufzeichnen umfasst, dass jenes Säugetier keine Gefäßerkrankung hat, wenn jenem Säugetier jene erhöhten Spiegel fehlen.

## Revendications

1. Procédé *in vitro* d'évaluation de la présence d'une maladie vasculaire chez un mammifère, dans lequel ledit procédé comprend la détermination du fait qu'un échantillon dudit mammifère contient ou non un niveau élevé de cellules progénitrices endothéliales CD34+/KDR+ circulantes exprimant un polypeptide lié aux os, dans lequel la présence dudit niveau élevé indique que ledit mammifère souffre d'une maladie vasculaire.

2. Procédé selon la revendication 1, dans lequel ledit mammifère est un être humain.

3. Procédé selon la revendication 1, dans lequel ladite maladie vasculaire est une athérosclérose coronarienne précoce.

4. Procédé selon la revendication 1, dans lequel ladite maladie vasculaire est une athérosclérose coronarienne tardive.

5. Procédé selon la revendication 1, dans lequel lesdites cellules progénitrices endothéliales CD34+/KDR+ circulantes sont des cellules progénitrices endothéliales CD133+/CD34+/KDR+ circulantes.

6. Procédé selon la revendication 1, dans lequel lesdites cellules progénitrices endothéliales CD34+/KDR+ circulantes sont des cellules progénitrices endothéliales CD133-/CD34+/KDR+ circulantes.

7. Procédé selon la revendication 1, dans lequel ledit procédé comprend la classification dudit mammifère comme souffrant d'une maladie vasculaire lorsque ledit mammifère présente ledit niveau élevé.

8. Procédé selon la revendication 1, dans lequel ledit procédé comprend la classification dudit mammifère comme ne souffrant pas d'une maladie vasculaire lorsque ledit mammifère ne présente pas ledit niveau élevé.

9. Procédé selon la revendication 1, dans lequel ledit procédé comprend l'enregistrement du fait que ledit mammifère souffre d'une maladie vasculaire lorsque ledit mammifère présente ledit niveau élevé.

10. Procédé selon la revendication 1, dans lequel ledit procédé comprend l'enregistrement du fait que ledit mammifère ne souffre pas d'une maladie vasculaire lorsque ledit mammifère ne présente pas ledit niveau élevé.
